# EUROPEAN PATENT APPLICATION

(11) **EP 1 302 103 A1**
(43) Date of publication of application: **16.04.2003**
(21) Application number: 01947834.6
(22) Date of filing: 04.07.2001
(51) Int. Cl.: A01K 61/00, C12M 3/00

(54) **METHOD OF CULTURING ZOOPLANKTON, APPARATUS FOR CULTURING BY THE SAME, AND CULTURE OBTAINED BY THE SAME**

(30) Priority: 06.07.2000 JP 2000205744
(71) Applicant: Nagase & Co., Ltd., Osaka-shi, Osaka 550-8668 (JP)
(72) Inventor: YOSHIMURA, Kenji, Munakata-shi, Fukuoka 811-3403 (JP); YOSHIMATSU, Takao, Onga-gun, Fukuoka 811-4204 (JP); TANAKA, Kenji, Fukuoka-shi, Fukuoka 815-0072 (JP); KITAJIMA, Chikara, Hiroshima-shi, Hiroshima 733-0853 (JP); TANAKA, Shozo, Fukuoka-shi, Fukuoka 812-0041 (JP); SATO, Nobumitsu, Kobe-shi, Hyogo 654-0122 (JP)
(74) Representative: Koepe, Gerd L., Dipl.-Chem.
(86) International application number: JP0105824
(87) International publication number: WO02003786

(57) **Abstract**

The present invention provides a method of culturing zooplankton that allows solids serving as feed for zooplankton to remain in a culture medium, while preventing accumulation of growth inhibitors of zooplankton and growth of bacteria, and an apparatus for culturing by the same. The culture method of the present invention includes removing a growth inhibitor of the zooplankton and/or a water-soluble nutrient for bacteria from a culture medium containing the zooplankton by a solid/liquid separation membrane with perfusion. The culture method of the present invention can produce a culture for use as feed for fishes or crustaceans containing at least 50,000 per ml of highly lively zooplanktons.

## Description

### Technical Field

The present invention relates to a method of culturing zooplankton and an apparatus for culturing by the same. More specifically, the present invention relates to a method of culturing zooplankton that is useful as feed for fishes, crustaceans or the like more efficiently and an apparatus for culturing by the same. The present invention also relates to a culture for use as feed useful as feed for fishes, crustaceans or the like.

### Background Art

In recent years, seafood such as red sea bream, tiger puffer, sweet fish, flatfish, or Peneus japonicus that has high market value and fishes that are viewed for pleasure such as tropical fish are grown using so-called artificial fish seeds that are raised after eggs are hatched. In the process of producing the artificial fish seeds effectively, zooplankton is necessary as feed.

Conventionally, zooplankton is cultured by feeding solid feeds such as Nannochloropsis, chlorella, yeast, or Tetraselmis in a water tank.

However, in cultivation in a water tank, in general, water-soluble substances such as ammonia, which is a metabolite, are accumulated in the culture medium as cultivation proceeds. It is also possible that other substances such as organic acids are accumulated in a culture medium. These substances are called growth inhibitors and are known to prevent growth of zooplankton by appearance of acute or chronic toxicity and adversely affect the cultivation itself.

Furthermore, since such cultivation in a water tank is generally performed in an open system, bacteria also tend to be grown by assimilating nutrients eluted in the culture medium. The grown bacteria compete with the zooplankton for oxygen in the culture medium. It is also known that some bacteria grown in the culture medium produce toxin and inhibit the growth of zooplankton.

Thus, in the cultivation in a water tank, the above-described factors interact in a complicated manner and make the growth of zooplankton unstable and limited.

Among zooplankton, Rotifera is an essential organism as feed in production processes of early fish seeds such as young fish in sea farming. Therefore, various methods for culturing Rotifera with a higher density are under development.

Japanese Laid-Open Patent Publication No. 9-172902 discloses a method of providing zooplankton such as rotifer continuously at a low cost as a continuous culture method for zooplankton. More specifically, feed and a culture medium are supplied to a culture tank containing rotifer and a culture medium containing rotifer is taken out to harvest the rotifer, so that the cultivation can be performed while the liquid surface in the culture tank can be kept constant.

However, in the above-method, there may be some limitations when used in actual sea farming. For example, when utilizing rotifer that has been grown by this method as a culture for use as feed, the amount of rotifer per unit volume is small. Therefore, a large-scale facility and/or equipment is necessary to produce a sufficient amount of rotifer required for feeding.

### Disclosure of Invention

The present invention relates to a method of culturing zooplankton with a high density. More specifically, the present invention has an object of providing a method of culturing zooplankton that can provide zooplankton with a high density by letting a solid serving as feed for zooplankton remain in a culture medium, using a solid/liquid separation membrane, while removing substances that inhibit the growth of the zooplankton in the culture medium and removing water-soluble nutrients associated with the growth of bacteria, and of providing an apparatus for culturing by the same. Furthermore, the present invention has another object of providing a culture for use as feed that contains zooplankton useful in cultivation of fishes, crustaceans or the like at a high density and can prevent reduction of a survival rate due to transportation.

The present invention provides a method of culturing zooplankton, comprising removing a growth inhibitor of the zooplankton and/or a water-soluble nutrient for bacteria from a culture medium containing the zooplankton by a solid/liquid separation membrane with perfusion.

In one preferable embodiment, the zooplankton is a multicellular organism.

In a more preferable embodiment, the multicellular organism is rotifer.

In one preferable embodiment, the growth inhibitor is ammonia or an organic acid, and the water-soluble nutrient is a water-soluble organic substance, mineral, vitamin, phosphoric acid, or ammonia.

The present invention also provides an apparatus for culturing zooplankton comprising:
a culture tank for storing a culture medium containing zooplankton;
a solid/liquid separation membrane provided in the culture tank;
means for removing a growth inhibitor of the zooplankton and/or a water-soluble nutrient for bacteria from the culture medium through the solid/liquid separation membrane; and
means for supplying feed for the zooplankton and a fresh culture medium to the culture tank.

In one preferable embodiment, the zooplankton is a multicellular organism.

In a more preferable embodiment, the multicellular organism is rotifer.

In one preferable embodiment, the pore size of the solid/liquid separation membrane is selected depending on a size of feed to be fed and a substance to be removed.

In a more preferable embodiment, a diffusing tube is further provided in the culture tank.

The present invention also provides a membrane cartridge to be attached to a culture tank for culturing zooplankton, comprising:
a plurality of solid/liquid separation membranes spaced away from each other by a constant distance by spacing means; and
means for gathering effluents discharged from discharge ports provided in the solid/liquid separation membranes.

In one preferable embodiment, the solid/liquid separation membrane is a flat separating membrane.

In one preferable embodiment, the spacing means and the solid/liquid separation membrane are fixed via a rod.

In a more preferable embodiment, the rod further includes holding means.

The present invention also provides a culture for use as feed containing 50,000 per ml to 200,000 per ml of lively zooplanktons.

In one preferable embodiment, the zooplankton is rotifer.

In a more preferable embodiment, the culture for use as feed contains 50,000 per ml to 100,000 per ml of lively zooplanktons.

In one preferable embodiment, the culture for use as feed contains 100,000 per ml to 200,000 per ml of lively zooplanktons.

The present invention also provides a method of producing a culture for use as feed, comprising removing a growth inhibitor of the zooplankton and/or a water-soluble nutrient for bacteria from a culture medium containing the lively zooplankton by a solid/liquid separation membrane with perfusion.

In one preferable embodiment, the culture for use as feed contains 50,000 per ml to 200,000 per ml of lively zooplanktons.

In one preferable embodiment, the zooplankton is rotifer.

### Brief Description of Drawings

Figure 1 is a schematic view illustrating an example of an apparatus for culturing zooplankton of the present invention.
Figure 2 shows views illustrating an example of a solid/liquid separation membrane used for the apparatus for culturing zooplankton of the present invention: Figure 2(a) is a schematic view of a flat solid/liquid separation membrane and Figure 2(b) is a schematic view of a cylindrical solid/liquid separation membrane.
Figure 3 is a perspective view illustrating an example of a membrane cartridge for culturing zooplankton employing a plurality of flat solid/liquid separation membranes.
Figure 4 is a schematic view illustrating the structure of the flat solid/liquid separation membrane used in the membrane cartridge shown in Figure 3.
Figure 5 is a side view of the membrane cartridge for culturing zooplankton of the present invention shown in Figure 3.
Figure 6 is a partially cutaway cross-sectional view of a culture apparatus employing the membrane cartridge of the present invention shown in Figure 3.
Figure 7 is a graph showing the amount of fed feed per culture day in Example 1.
Figure 8 is a graph showing a change in the density of rotifer in the culture medium in Example 1.
Figure 9 is a graph showing a change in the ammonia concentration in the culture medium in Example 1.
Figure 10 is a graph showing a change in the concentration of dissolved oxygen in the culture medium in Example 1.

### Best Mode for Carrying Out the Invention

According to the method of the present invention, zooplankton can be obtained at a high density by letting solids serving as feed for zooplankton remain in a culture medium, using a solid/liquid separation membrane, while removing substances that inhibit the growth of the zooplankton in the culture medium and removing water-soluble nutrients associated with the growth of bacteria.

The zooplankton used in the present invention is an organism for use as feed for fishes or crustaceans. Examples of the zooplankton include saltwater, freshwater and brackish water zooplankton such as Rotifera (rotifer); Acaltio; Copepoda such as Tigriopus; Daphnia pulex; and Aritemia In the present invention, it is preferable that the zooplankton is multicellular organisms. Multicellular organisms generally take in solids such as micro-suspended substances in water typified by micro-seaweeds as nutrients. Therefore, even if water-soluble nutrients as described later are removed from the culture medium, solids remain in the culture medium, so that sufficient growth can be expected.

In the present invention, Rotifera can be used most preferably as the zooplankton. Examples of Rotifera include the L-type rotifer (Brachionus plicatilis) and the S-type rotifer (B. rotundiformis). In the present invention, both the L-type rotifer and the S-type rotifer can be used preferably. Furthermore, a mixture of the L-type rotifer and the S-type rotifer can be used. The L-type rotifer that can be used in the present invention generally has an armor length of 150 µm to 300 µm, preferably 250 µm to 300 µm, when it is grown up. The S-type rotifer that can be used in the present invention generally has an armor length of 100 µm to 230 µm, preferably 180 µm to 200 µm, when it is grown up.

As the culture medium used in the present invention, liquids such as seawater, freshwater and brackish water can be used. The kind of the culture medium depends on the kind of zooplankton to be cultured. The culture medium suitable for the kind of zooplankton to be cultured can be selected as appropriate by those skilled in the art.

In the present invention, zooplankton is cultured in such a culture medium. There is no particular limitation regarding cultivation conditions, and appropriate conditions can be selected in accordance with the zooplankton to be cultured by those skilled in the art.

Substances that inhibit the growth of zooplankton (hereinafter, referred to as "growth inhibitors") refer to substances that affect adversely the zooplankton in the culture medium. These substances are eliminated to the culture medium as a result of the metabolism of zooplankton and/or other coexisting microorganisms, and accumulated therein. Examples of the growth inhibitors include ammonia, in particular, undissociated ammonia (NH₃), and organic acids, which are produced during cultivation of zooplankton. Therefore, in the method of the present invention, it is preferable that these growth inhibitors are not contained to the possible extent, or even if they are contained, the amount thereof is kept low.

From such a viewpoint, in the method of the present invention, the acceptable amount of the growth inhibitors in the culture medium is, for example, preferably 40 ppm or less, and more preferably 10 ppm or less in the case of undissociated ammonia. In the case of dissociated ammonia (NH₄⁺), the toxicity with respect to the zooplankton is about 1/10 of that of undissociated ammonia, so that the concentration of dissociated ammonia in the culture medium that can be actually measured is preferably 400 ppm or less, and more preferably 100 ppm or less.

The water-soluble nutritive substances associated with the growth of bacteria (hereinafter, referred to as "water-soluble nutrients") refer to substances that can be nutrient components for bacteria, and can be, for example, water-soluble organic substances, vitamins, minerals, phosphoric acid, ammonia or the like.

Feed for zooplankton used in the present invention can be selected as appropriate in accordance with the zooplankton to be cultured. For example, chlorella (freshwater chlorella (size of 3 µm to 10 µm), marine chlorella (size of 2 µm to 6 µm), yeast (size of 5 µm to 10 µm), Tetraselmis (size of 10 µm to 20 µm), Phaeodactylum tricornutum (size of 3 µm to 30 µm) can be used. There is no particular limitation regarding the feeds for zooplankton contained in the culture medium, as long as they do not affect the growth and the survival of the zooplankton. The amount of the feed for the zooplankton is not limited to a specific value, because it depends on the kind of the zooplankton to be cultured. The amount of the feed can be selected as appropriate depending on the kind of the zooplankton to be cultured by those skilled in the art.

In the culture method of the present invention, it is possible to culture zooplankton at a high concentration in a culture medium. For example, in the case of culturing Rotifera as described above, the number of Rotifera contained in the culture medium is at least 50,000, preferably 50,000 to 200,000, more preferably 70,000 to 200,000, even more preferably 80,000 to 180,000 per ml.

More specifically, the cultivation of zooplankton can be performed with a culture apparatus of the present invention in the following manner.

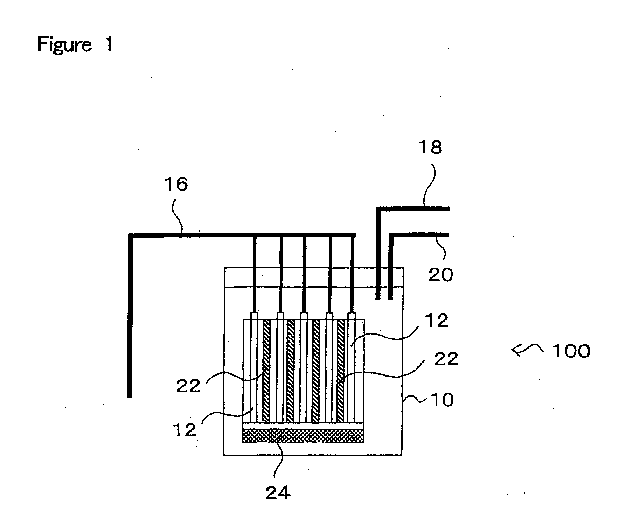

Figure 1 is a schematic and conceptual view for illustrating an example of an apparatus for culturing zooplankton of the present invention. A culture apparatus of the present invention 100 includes a culture tank 10 for storing a culture medium containing zooplankton, a solid/liquid separation membrane 12 attached to the culture tank 10, a discharge tube 16 in communication with the solid/liquid separation membrane 12, and a feeding tube 18 and a liquid supplying tube 20 for supplying fresh feed and fresh culture medium.

There is no particular limitation regarding the material of the culture tank 10 used in the present invention. A culture tank used in ordinary cultivation of zooplankton can be used. There is no limitation regarding the volume of the culture tank 10, and a desired volume can be selected by those skilled in the art. The culture apparatus of the present invention can culture zooplankton at a higher density than a conventional apparatus. Therefore, it is not necessarily to select a culture tank having a volume equal to that of a conventionally used tank. On the other hand, when a culture tank having a volume equal to that of a conventionally used tank is used, the number of zooplankton in the culture obtained increases drastically. In the culture tank 10, various control devices necessary for growth of zooplankton, such as a thermostat for keeping the temperature constant, a heater, a cooling apparatus, a computer for controlling these devices (neither shown) may be attached to suitable positions inside or outside of the culture tank.

The solid/liquid separation membrane 12 is attached so as to be immersed in the culture medium containing zooplankton in the culture tank 10.

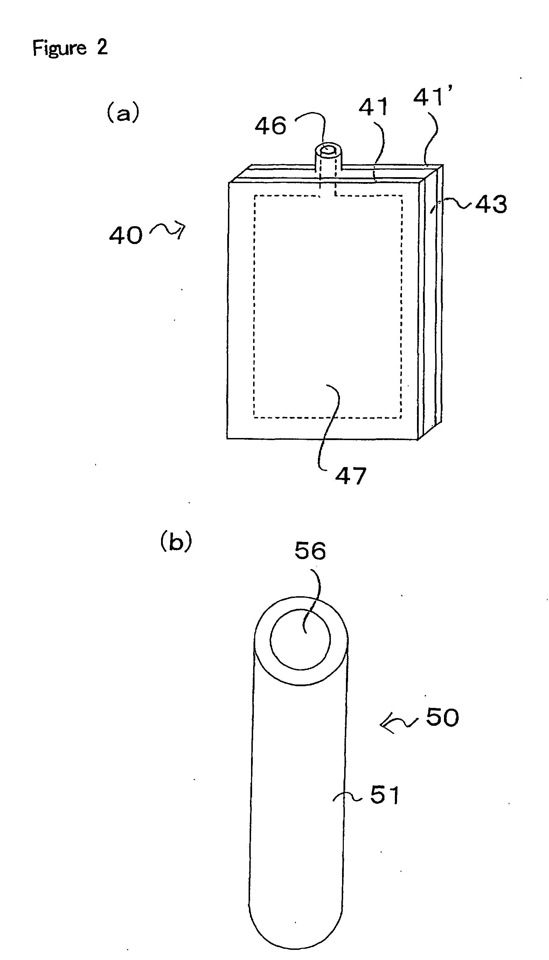

Examples of the solid/liquid separation membrane 12 include a flat membrane 40 (Figure 2(a)) and a cylindrical membrane 50 (Figure 2(b)), as shown in Figure 2.

As shown in Figure 2(a), the flat solid/liquid separation membrane 40 generally includes two flat membrane members 41 and 41' and a frame 43. The peripheries of both the flat membrane members 41 and 41' are attached completely to the frame 43, which defines a space 47 inside the flat solid/liquid separation membrane 40. This space 47 is in communication with a discharge port 46 provided in the frame 43. The discharge port 46 is connected to a discharge tube 16 in the culture apparatus of the present invention. In this flat solid/liquid separation membrane, the membrane member may be provided only on one surface of the frame and the other surface may be covered with a resin plate.

As shown in Figure 2(b), the cylindrical solid/liquid separation membrane 50 is formed of a hollow membrane member 51. Both end portions of the cylindrical solid/liquid separation membrane 50 may be connected to the discharge tube 16 in the culture apparatus of the present invention to serve as discharge ports. Alternatively, one end portion may be capped, and only the other end portion may be connected to the discharge tube in the culture apparatus of the present invention to serve as a discharge port 56.

The material of these membrane members used in the solid/liquid separation membrane is not limited to any specific material but can be selected as appropriate by those skilled in the art. The membrane member used in the solid/liquid separation membrane includes a large number of pores on its surface. The pore size is not limited to any specific value but can be selected as appropriate by those skilled in the art, depending on feeds to be fed and substances to be removed, the size of the zooplankton to be cultured or the like. It is sufficient that the solid/liquid separation membrane employing a membrane member having a predetermined pore size does not allow zooplankton or feed for the zooplankton (e.g., freshwater chlorella (size of 3 µm to 10 µm)) to permeate through it, but allows growth inhibitors and/or water-soluble nutrients, and suspended substances in the culture medium (e.g., excrements of zooplankton and residues of feed) to permeate through it. For example, for cultivation of rotifer, the pore size is preferably about 0.2 µm to about 2 µm, more preferably about 0.4 µm to about 1.5 µm, most preferably 0.4 µm to 1.0 µm. Contaminated microorganisms such as bacteria (generally, about 1 µm or less) may permeate through the membrane, depending on the pore size of the membrane member in the selected solid/liquid separation membrane.

Those skilled in the art can select and use an appropriate solid/liquid separation membrane including the membrane member having such a material and pores. Examples of the material of the membrane member include synthetic polymer films (membrane filters), ceramic films, and metal films.

Examples of the growth inhibitors to be permeated through the solid/liquid separation membrane and be removed include ammonia and organic acids produced during cultivation, as described above. Examples of the water-soluble nutrients that can permeate through the solid/liquid separation membrane include water-soluble organic substances, vitamins, minerals, phosphoric acid, and ammonia, as described above. The solid/liquid separation membrane allows these growth inhibitors and water-soluble nutrients to permeate through it, so that only these substances can be removed from the culture medium while the zooplankton and its feed are allowed to remain.

Referring to Figure 1 again, in the culture apparatus 100 of the present invention, there is no particular limitation regarding the effective surface area and the number of the solid/liquid separation membrane 12 arranged in the culture tank 10. The effective surface area of the solid/liquid separation membrane necessary to remove the growth inhibitors and the water-soluble nutrients from the culture medium more efficiently can be set as appropriate in accordance with a desired filtration flow rate. Therefore, it is preferable to provide a plurality of solid/liquid separation membranes in the culture tank. In the culture apparatus of the present invention, when a plurality of solid/liquid separation membranes are provided in the culture tank, it is preferable that the solid/liquid separation membranes are spaced away from each other by a constant interval. Thus, the filtration efficiency of the respective solid/liquid separation membranes is also constant, so that they can be replaced by new ones at the same time.

In the present invention, as shown in Figure 1, apart from the solid/liquid separation membrane 12, a filter 22 may be included in the culture tank 10. The filter 22 has a pore size that is larger than that of the solid/liquid separation membrane 12. The filter has the void ratio depending on the kind of zooplankton to be cultured, so that it serves as an object to which microorganism flocks and biofilms accumulated in the culture medium are attached or adsorbed. The filter 22 prevents the microorganism flocks or biofilms from being attached directly to the solid/liquid separation membrane 12 and thus prevents fouling (clogging). There is no limitation regarding the kind of the filter 22, and for example, Vilene mat manufactured by JAPAN VILENE COMPANY, LTD. can be used.

Furthermore, in the present invention, it is preferable that a diffusing tube 24 is provided on the bottom surface in the culture tank 10, as shown in Figure 1. The diffusing tube 24 is connected to an air pump (not shown). The diffusing tube 24 makes it possible to supply fresh air useful in the cultivation of zooplankton to the culture medium in the culture tank 10 as appropriate, set the oxygen concentration in the culture medium constantly high, and prevent fouling. It is preferable that the amount of oxygen dissolved in the culture medium is set to a concentration of at least 2 ppm.

In Figure 1, the diffusing tube 24 is an integral part of the solid/liquid separation membrane 12 at its lower end, but the present invention is not limited thereto. The diffusing tube 24 can be separated from the solid/liquid separation membrane 12 and arranged on the bottom of the culture tank 10.

The discharge tube 16 used in the present invention is in communication with a suction apparatus (not shown) such as a suction pump and draws a predetermined amount of the culture medium in the culture tank 10 through the solid/liquid separation membrane 12. The amount of the culture medium to be drawn can be set as appropriate, depending on the volume of the culture tank 12, the kind of zooplankton to be cultured, the culture conditions or the like by those skilled in the art.

When as the membrane member constituting the solid/liquid separation membrane, for example, a ceramic film is used, the suction of the culture medium can be interrupted temporarily at a predetermined time interval, and the discharge tube can be used as a diffusing tube for supplying air. Thus, fresh air can be supplied into the culture medium, and fouling on the surface of the ceramic film on the side of the culture medium can be prevented. Specific examples of the ceramic film apparatus that can be used include KUBOTA FILCERA® (KUBOTA Corporation).

On the other hand, the feeding tube 18 and the liquid supplying tube 20 are also in communication with the culture tank 10 and can supply feed and fresh culture medium through some means (not shown) such as a liquid supplying pump, depending on the amount of the culture medium discharged from the discharge tube 16.

Thus, when the apparatus of the present invention is used, the growth inhibitors and the water-soluble nutrients in the culture medium that are toxic to the cultivation of zooplankton are removed, and the zooplankton can be cultured efficiently.

In the present invention, it is more preferable that the solid/liquid separation membrane 12 has a structure in which a plurality of solid/liquid separation membranes 12 are formed into an integral unit. Examples of the integral structure include a cartridge and a module. This structure allows periodic replacement and cleaning, and prevents fouling in the solid/liquid separation membrane 12 and the filter 22.

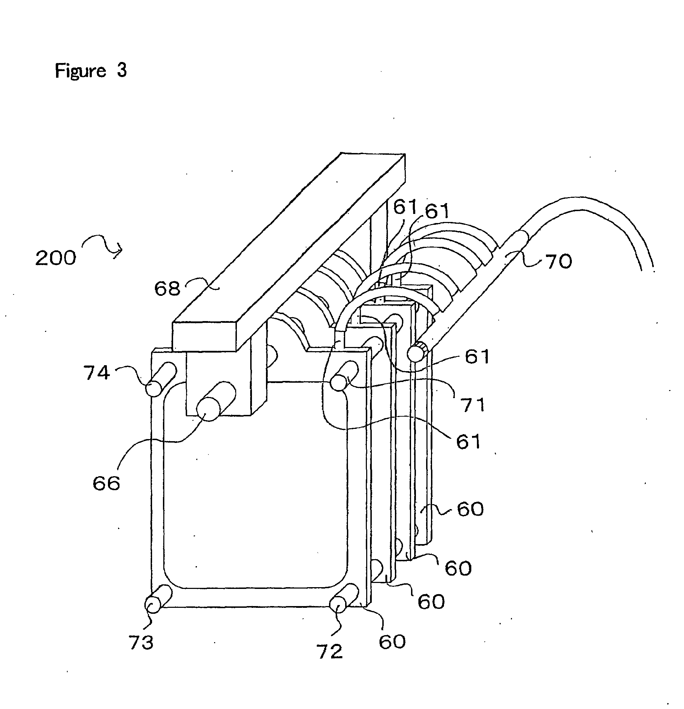

Figure 3 is a perspective view for illustrating an example of a membrane cartridge for culturing zooplankton using a plurality of flat solid/liquid separation membranes. A membrane cartridge 200 of the present invention includes flat solid/liquid separation membranes 60 (four membranes in Figure 3), a main rod 66 and sub rods 71, 72, 73, and 74 for supporting the membranes, and a handle 68 that is penetrated by the main rod 66, for facilitating mounting and movement of the cartridge. All the discharge ports 61 provided in the flat solid/liquid separation membranes 60 are gathered to an adaptor 70 through a silicone tube or the like. In general, one end of the adaptor 70 is sealed by a cap, and the other end is connected to a suction pump that is not shown in the drawing through a discharge tube such as a silicone tube, a plastic tube or a rubber tube.

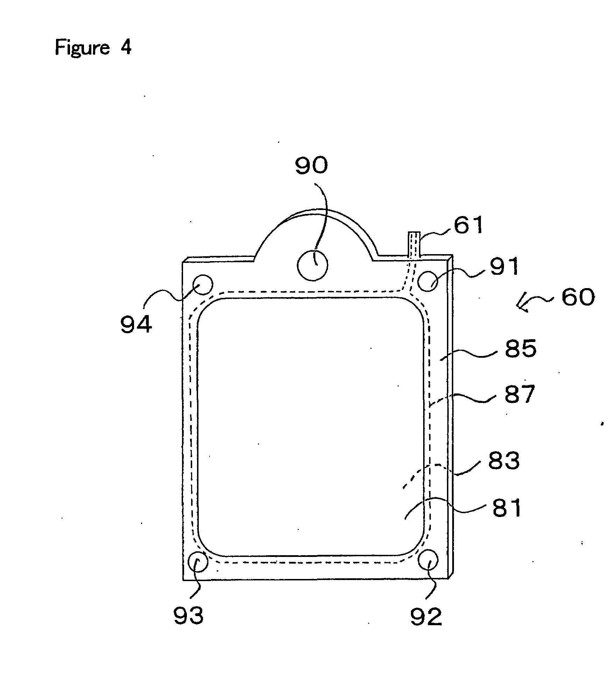

Figure 4 is a schematic view for illustrating the structure of the flat solid/liquid separation membrane 60 used in the above-described membrane cartridge. In the flat solid/liquid separation membrane 60, the peripheries of two membrane members 81 and 83 made of synthetic polymer films are surrounded by a rectangular frame 85 made of a resin, and a space 87 is formed inside. The space 87 is in communication with the discharge port 61. In the frame 85, a main hole 90 for passing the main rod through is provided in the upper central portion of the frame, and sub holes 91, 92, 93, and 94 for passing the sub rods through are provided in the vicinity of the respective corners. The main hole 90 and the sub holes 91, 92, 93 and 94 are not in communication with the space 87. This structure of the flat solid/liquid separation membrane 60 makes it possible that filtrate entered the space 87 through the membrane members 81 and 83 can be passed only through the discharge port 61.

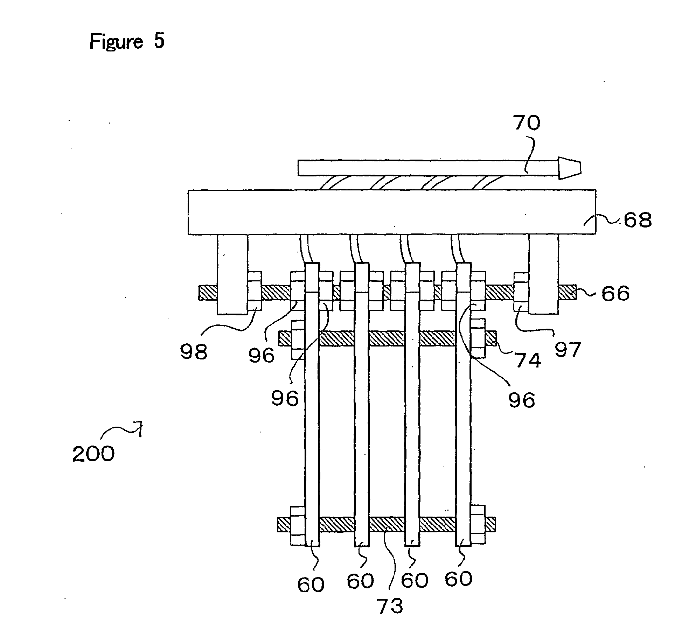

Figure 5 is a side view of the membrane cartridge 200 for culturing zooplankton of the present invention shown in Figure 3. The solid/liquid separation membranes 60 are fixed while being spaced away from each other by a constant distance by a plurality of nuts 96 that are penetrated by the main rod 66. The handle 68 is also fixed with other nuts 97 and 98 that previously have been penetrated by the main rod 66.

Thus, in the membrane cartridge of the present invention, the plurality of solid/liquid separation membranes can be fixed while being spaced away from each other by a constant distance, and the membrane cartridge can be removed from the culture tank easily by holding the handle.

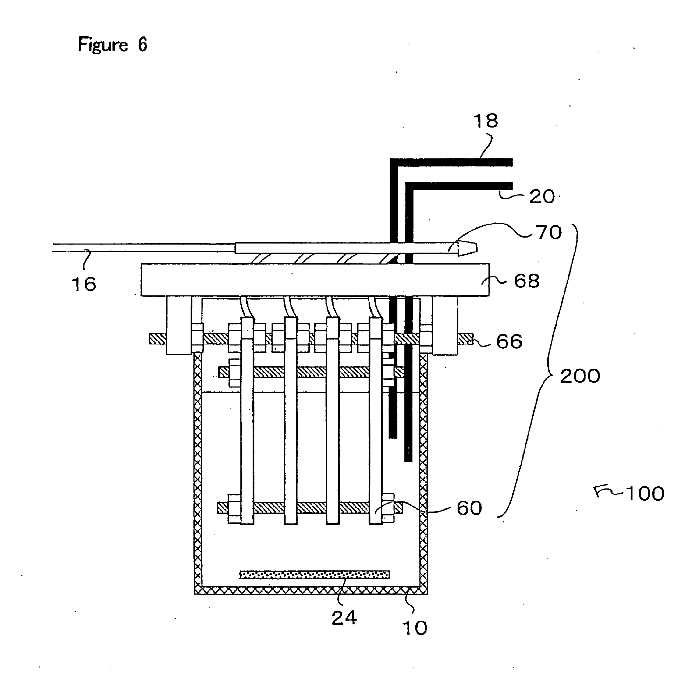

It is preferable that the membrane cartridge 200 of the present invention is arranged with respect to the culture tank 10 such that the handle 68 is mounted on the upper portion of the culture tank 10, as shown in Figure 6. When the depth of the culture tank 10 is larger than the length of the solid/liquid separation membrane 60, such a manner of mounting makes the solid/liquid separation membrane 60 suspended in the culture tank 10. As a result, it is avoided that the solid/liquid separation membrane 60 is in contact with the culture tank 10 or the diffusing tube 24, so that the possibility of damage of the culture tank, the diffusing tube and/or the solid/liquid separation membrane can be prevented.

When zooplankton is cultured using the membrane cartridge of the present invention, microorganism flocks and biofilms accumulated in the culture medium may be attached or adsorbed onto the surface of the solid/liquid separation membrane in the membrane cartridge as time passes. In this case, they can be removed by holding the handle and taking out the membrane cartridge, immersing it, for example, in a sodium hypochlorite aqueous solution, then adding sodium thiosulfate, and thereafter washing it with water. On the other hand, another membrane cartridge can be mounted on the culture tank.

Since the culture obtained by the culture method of the present invention contains zooplankton at a high density, it can be utilized as a culture for use as feed. In the culture for use as feed of the present invention, the zooplankton can be obtained only by culture means (e.g., the above-described culture means) without using regular concentration means. Most preferably, the culture for use as feed of the present invention contains "lively rotifer" as the zooplankton. As used in the specification, "lively rotifer" refers to living rotifer, and does not contain dead rotifer. It is preferable that the culture for use as feed of the present invention is grown up to the state where it is useful as feed for cultured organisms such as fishes, crustaceans or the like and are highly vital. The culture for use as feed may contain both the L-type rotifer and the S-type rotifer.

The culture for use as feed of the present invention contains zooplankton at a high density. For example, when the culture for use as feed of the present invention contains the above-described lively rotifer as the zooplankton, the number of the lively rotifer contained in the culture is at least 50,000 per ml, preferably 50,000 to 200,000, more preferably 70,000 to 200,000, and even more preferably 80,000 to 180,000 per ml. It is preferable in the case of the L-type rotifer that the number is 50,000 to 100,000 per ml of the culture, and it is preferable in the case of the S-type rotifer that the number is 100,000 to 200,000 per ml. When the number is less than 50,000 per ml, this causes disadvantages in terms of low cost and space saving because the volume is large when the culture is used as feed as it is.

The culture for use as feed of the present invention also includes the culture medium used when zooplankton is cultured, in addition to the zooplankton. This culture medium may consist mainly of a liquid such as seawater, freshwater or brackish water, and may contain a very small amount of growth inhibitors and/or water-soluble nutrients derived from the cultivation.

The culture for use as feed of the present invention also may contain feed (e.g., freshwater chlorella, marine chlorella) of zooplankton used when the zooplankton is cultured. There is no limitation regarding the feed of the zooplankton contained in the culture, as long as it does not affect the viability of the zooplankton in the culture.

Hereinafter, examples of the present invention will be described. The present invention is not limited by these examples.

### <Example 1>

Hereinafter, the present invention will be described by way of examples with S-type marine small rotifer (hereinafter, referred to as "rotifer"), which is one of zooplankton.

A section (experiment section) in which the apparatus shown in Figure 1 (solid/liquid separation membrane: KUBOTA Corporation, submerged membrane cartridge H3-203; pore size: 0.4 µm; effective area: 0.11 m²) was provided and a section (control section) obtained by removing the solid/liquid separation membrane from the apparatus shown in Figure 1 were provided in a 20 liter water tank. Rotifer was inoculated at a concentration of 3000 per ml to both the sections, and batch culture was performed. The culture temperature was 32°C in both the sections, and oxygen gas aeration was performed at 3 liters/min. For the feeds, commercially available concentrated freshwater chlorella was used and fed in the same amount to both the experiment section and the control section every day. In the experiment section, 20 to 40 liters (about 100 to 200 % by volume of the amount of the culture medium) of the culture medium were drawn out and fresh seawater was added.

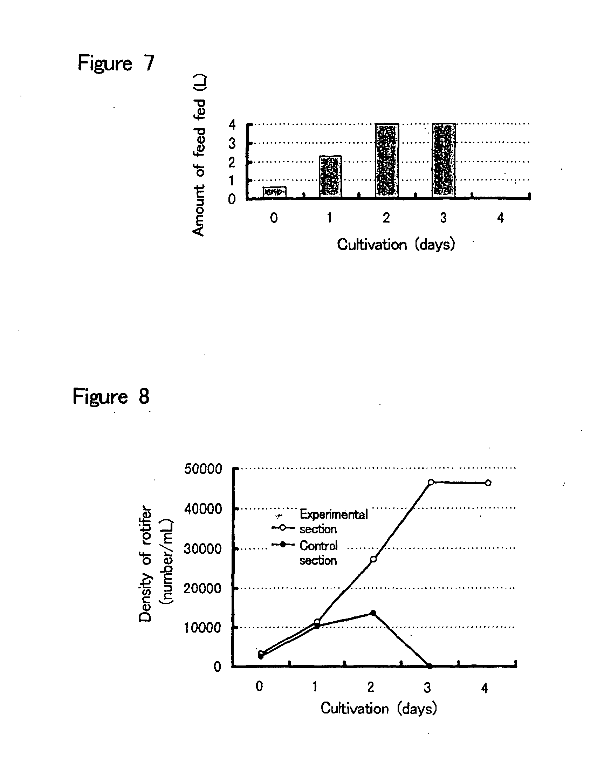

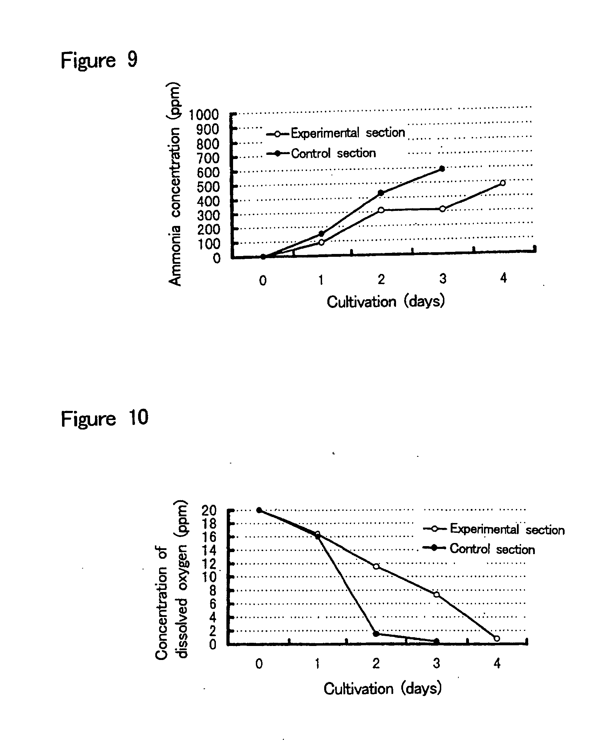

It is known that in the cultivation of rotifer, the lack of oxygen and an increase of ammonia concentration inhibit the growth. Figure 7 shows the amount of the feed fed per day, and Figure 8 shows a change in the density of rotifer in the culture medium in accordance therewith. Furthermore, Figure 9 shows a change in the ammonia concentration in the culture medium obtained by this cultivation, and Figure 10 shows a change in the concentration of dissolved oxygen. The density (number/ml) of lively rotifer in the culture medium was measured using a stereoscopic microscope. The ammonia concentration in the culture medium was measured using an ammonia meter T1-9001 (manufactured by Tokushige Kagaku). The concentration of dissolved oxygen was measured with a dissolved oxygen meter (Model 58 manufactured by YSI).

In the control section, the rotifer density reached 13500/ml, which was the peak, on day 2 of the cultivation. On day 3 of the cultivation, the ammonia concentration was about 600 ppm, and the concentration of dissolved oxygen was substantially 0 ppm, and rotifer was substantially dead. On the other hand, in the experiment section, the ammonia concentration was kept low for a long time and the concentration of dissolved oxygen was kept high, compared to the control section. On day 3 of the cultivation, the rotifer density reached 46000/ml. In the experiment section, the rotifer density was about three times the density in the control section, and the viability of the rotifer was also good.

### <Example 2>

Using the apparatus shown in Figure 1 (solid/liquid separation membrane: KUBOTA Corporation, submerged membrane cartridge H3-203; pore size: 0.4 µm; effective area: 0.11 m²) in a 40 liter water tank, rotifer was inoculated at a concentration of 9200 per ml to the culture tank, and cultivation was performed with perfusion. For the feed, concentrated freshwater chlorella (manufactured by Nissinsya) was used and fed in an amount shown in Table 1 every day. As the perfusion condition, 160 to 200 liters (about 400 to 500 % of the amount of the culture medium) of the culture medium were drawn out from the culture tank and fresh seawater was added in the same amount. The culture temperature was 32°C, and oxygen gas aeration was performed at 6 liters/min. This cultivation was performed for five days.

Every day this culture medium was taken out, and the density of the lively rotifer and the ammonia concentration in the culture medium were measured. Table 1 shows the density of the lively rotifer in the culture obtained and Table 2 shows the ammonia concentration in the culture.

### <Example 3>

Rotifer was cultured in the same conditions as in Example 2, except that rotifer was inoculated at a concentration of 14000 per ml. Table 1 shows the density of the lively rotifer in the culture obtained.

### <Example 4>

Rotifer was cultivated in the same conditions as in Example 2, except that rotifer was inoculated at a concentration of 14200 per ml. Table 1 shows the density of the lively rotifer in the culture obtained.

### <Comparative Example 1>

Rotifer was cultured for five days in the same conditions as in Example 2, except that in the culture apparatus used in Example 2, rotifer was inoculated at a concentration of 11200 per ml to the culture tank, and cultivation was performed under batch conditions without performing perfusion nor using the solid/liquid separation membrane. Table 1 shows the density of the lively rotifer in the culture obtained, and Table 2 shows the ammonia concentration in the culture.

**Table 1**

| Lively rotifer density in a culture | | | | | | |
|---|---|---|---|---|---|---|
| | Cultivation | | | | | |
| | Day 0 | Day 1 | Day 2 | Day 3 | Day 4 | Day 5 |
| Amount of chlorella fed (L) | 3000 | 3000 | 6000 | 10000 | 0 | 0 |
| Experiment 2 | 9200 | 11200 | 33600 | 55200 | 78400 | 176000 |
| Experiment 3 | 14000 | 9800 | 26400 | 52400 | 87200 | 160000 |
| Experiment 4 | 14200 | 14600 | 28400 | 59200 | 84000 | 162000 |
| Com.Ex.1 | 12000 | 12400 | 30800 | 32000 | 0 | 0 |
| Average values of three samples are shown Unit: number/ml | | | | | | |

**Table 2**

| Ammonia concentration in a culture | | | | | | |
|---|---|---|---|---|---|---|
| | Cultivation | | | | | |
| | Day 0 | Day 1 | Day 2 | Day 3 | Day 4 | Day 5 |
| Experiment 2 | 2.4 | 19 | 160 | 320 | 310 | 86 |
| Com.Ex.1 | 2.3 | 39 | 440 | 860 | 690 | not measured |
| Unit: ppm | | | | | | |

As shown in Table 1, in all of the cultures obtained in Examples 2 to 4, rotifer was cultured up to a concentration of about 200,000 per ml on day 5 of the cultivation, and a high density rotifer culture was obtained. On the other hand, in Comparative Example 1, rotifer in the culture tank was dead by day 4, and a high density rotifer culture was not obtained.

When comparing the ammonia concentration in the culture tank of Example 2 to that of Comparative Example 1, the ammonia concentration in the tank of the culture obtained in Example 2 did not increase very much, regardless of the period of the cultivation. On the other hand, the ammonia concentration in the tank of the culture obtained in Comparative Example 1 increased as the culture days passed, and adversely affected the cultivation of rotifer.

Furthermore, when the rotifer of the culture mediums on day 5 obtained in Examples 2 to 4 were observed, it was found that the vitality of the rotifer was good.

### Industrial Applicability

According to the present invention, the concentration of ammonia, which is a growth inhibitor, in the culture medium can be suppressed to a low level, and the concentration of dissolved oxygen can be kept high. Thus, desired zooplankton can be cultured at a higher density. In other words, livelier zooplankton can be provided as a culture for use as feed for fishes or crustaceans. Furthermore, zooplankton is contained at a higher density in a predetermined volume, so that production efficiency is good, and cost performance is expected to improve. In the present invention, the above effect is expected to be increased further by increasing the volume of the culture tank and the amount of perfusion involved in discharge and supply of the culture medium.

## Claims

1. A method of culturing zooplankton, comprising removing a growth inhibitor of the zooplankton and/or a water-soluble nutrient for bacteria from a culture medium containing the zooplankton by a solid/liquid separation membrane with perfusion.

2. The method according to claim 1, wherein the zooplankton is a multicellular organism.

3. The method according to claim 2, wherein the multicellular organism is rotifer.

4. The method according to any one of claims 1 to 3, wherein the growth inhibitor is ammonia or an organic acid, and the water-soluble nutrient is a water-soluble organic substance, vitamin, mineral, phosphoric acid, or ammonia.

5. An apparatus for culturing zooplankton comprising:
a culture tank for storing a culture medium containing zooplankton;
a solid/liquid separation membrane provided in the culture tank;
means for removing a growth inhibitor of the zooplankton and/or a water-soluble nutrient for bacteria from the culture medium through the solid/liquid separation membrane; and
means for supplying feed for the zooplankton and a fresh culture medium to the culture tank.

6. The culture apparatus according to claim 5, wherein the zooplankton is a multicellular organism.

7. The culture apparatus according to claim 6, wherein the multicellular organism is rotifer.

8. The culture apparatus according to any one of claims 5 to 7, wherein a pore size of the solid/liquid separation membrane is selected depending on a size of feed to be fed and a substance to be removed.

9. The culture apparatus according to any one of claims 5 to 8, wherein a diffusing tube is further provided in the culture tank.

10. A membrane cartridge to be attached to a culture tank for culturing zooplankton, comprising:
a plurality of solid/liquid separation membranes spaced away from each other by a constant distance by spacing means; and
means for gathering effluents discharged from discharge ports provided in the solid/liquid separation membranes.

11. The membrane cartridge according to claim 10, wherein the solid/liquid separation membrane is a flat separation membrane.

12. The membrane cartridge according to claim 10 or 11, wherein the spacing means and the solid/liquid separation membrane are fixed via a rod.

13. The membrane cartridge according to claim 12, wherein the rod further includes holding means.

14. A culture for use as feed containing 50,000 per ml to 200,000 per ml of lively zooplanktons.

15. The culture for use as feed according to claim 14, wherein the zooplankton is rotifer.

16. The culture for use as feed according to claim 14 or 15, containing 50,000 per ml to 100,000 per ml of lively zooplanktons.

17. The culture for use as feed according to claim 14 or 15, containing 100,000 per ml to 200,000 per ml of lively zooplanktons.

18. A method of producing a culture for use as feed, comprising:
removing a growth inhibitor of the zooplankton and/or a water-soluble nutrient for bacteria from a culture medium containing the lively zooplankton by a solid/liquid separation membrane with perfusion.

19. The method according to claim 18, wherein the culture for use as feed contains 50,000 per ml to 200,000 per ml of lively zooplanktons.

20. The method according to claim 18 or 19, wherein the zooplankton is rotifer.
